# EUROPÄISCHE PATENTSCHRIFT

(11) **EP 2 528 552 B1**
(45) Veröffentlichungstag und Bekanntmachung des Hinweises auf die Patenterteilung: **28.03.2018**
(21) Anmeldenummer: 11702584.1
(22) Anmeldetag: 28.01.2011
(51) Int. Cl.: A61F 2/958, A61M 25/00, A61F 2/966

(54) **MEDIZINISCHER KATHETER UND MEDIZINISCHES SYSTEM MIT EINEM DERARTIGEN KATHETER**
MEDICAL CATHETER AND MEDICAL SYSTEM COMPRISING SUCH A CATHETER
CATHÉTER MÉDICAL ET SYSTÈME MÉDICAL COMPORTANT UN CATHÉTER DE CE TYPE

(30) Priorität: 29.01.2010 DE 102010006187
(43) Veröffentlichungstag der Anmeldung: 05.12.2012
(73) Patentinhaber: Acandis GmbH & Co. KG, 76327 Pfinztal (DE)
(72) Erfinder: BÜCHERT, Michael, 70193 Stuttgart (DE)
(74) Vertreter: Kilchert, Jochen
(86) Internationale Anmeldenummer: PCT/EP2011/000410
(87) Internationale Veröffentlichungsnummer: WO 2011/092030

(56) Entgegenhaltungen:
- EP-A1- 0 839 504
- EP-A1- 1 518 515
- WO-A1-01/78627
- WO-A1-2009/100210
- WO-A2-99/24105
- WO-A2-2004/087006
- WO-A2-2008/036314
- US-A- 5 662 703
- US-A1- 2007 225 789
- US-A1- 2009 319 017
- US-A1- 2011 077 619

## Beschreibung

Die Erfindung betrifft einen medizinischen Katheter zum Zuführen eines selbstexpandierbaren, nicht-vorgeladenen Stents in ein Körperhohlorgan.

Periphere Gefäßverschlüsse werden üblicherweise mit Stents geöffnet, die auf einen Ballonkatheter montiert sind und nach Positionierung und Inflation des Ballons im verengten Segment expandiert werden. Der Nachteil bei diesen Stents besteht darin, dass leichte Schläge bzw. externe Druckeinwirkungen, beispielsweise bei Unfällen bzw. im Sport auf die vom Stent gestützte Gefäßstelle zu einem Wiederverschluss führen können, da sich der Stents nicht erneut ausdehnt. Ferner ist es üblich, durch angioplastische Verfahren, wie bspw. durch Ballondilatation krankhaft verengte Blutgefäße aufzudehnen, wobei sich ein an einem Gefäßkatheter angebrachter Ballon an der verengten Stelle unter hohem Druck entfaltet. Diese Verfahren verhelfen zu einer spontanen Besserung, die jedoch nicht immer von langer Dauer ist.

Aus diesen Gründen gewinnen selbstexpandierbare Stents aus Formgedächtniswerkstoffen, beispielsweise Nitinol-Stents, im Peripheriebereich (Beine/Arme), aber auch in anderen Körperbereichen stark an Bedeutung und verzeichnen große Zuwachsraten. Selbstexpandierbare Stents werden mit Hilfe von Kathetern implantiert, bei denen der gecrimpte Stent in der Katheterleitung verriegelt ist und sich beim Entlassen aus der Katheterleitung selbsttätig entfaltet. Ein solcher Katheter ist in EP 1 374 801 A1 beschrieben.

Da die vom selbstexpandierbaren Stent erzeugbaren Radialkräfte oftmals nicht für eine Weitung der Stenose ausreichen, werden zusätzlich Ballonkatheter eingesetzt, um eine Vordilatation der Stenose zu erreichen, bevor der Stent platziert wird. Gegebenenfalls wird nach dem Setzen des Stents erneut ein Ballonkatheter verwendet, um den im Gefäß platzierten Stent nachzuweiten. Die sichere Stützung des Gefäßes übernimmt der selbstexpandierbare Stent alleine. Externe Druckeinwirkungen auf die abgestützte Gefäßstelle werden vom Stent kompensiert, der sich selbstständig wieder aufweitet, so dass die Gefahr eines Wiederverschlusses beim Sport, bei Stürzen, bei Unfällen oder anderen Umständen, bei denen es zu einer externen Druckeinwirkung auf die abgestützte Gefäßstelle kommt, verringert wird.

Das herkömmliche Verfahren zum Implantieren von selbstexpandierbaren Stents umfasst die folgenden Schritte, nachdem ein Gefäßzugang in der Leiste gelegt und ein Führungsdraht bis in eine Position distal von der Stenose eingeführt wurde:
1. Einführen eines ersten Ballonkatheters über den Führungsdraht in die Stenose;
2. Inflatieren des Ballons zur Weitung der Stenose und anschließendes Deflatieren;
3. Entfernen des ersten Ballonkatheters;
4. Einführen eines Stent-Delivery-Katheters in die Stenose;
5. Freisetzen des Stents;
6. Entfernen des Stent-Delivery-Katheters;
7. Einführen eines zweiten Ballonkatheters in die Stenose;
8. Inflatieren des Ballons zur Weitung des Stents in der Stenose und anschließendes Deflatieren und
9. Entfernen des zweiten Ballonkatheters.

Das vorstehend beschriebene bekannte Verfahren ist umständlich, zeitaufwändig und anfällig für Fehler. Ferner besteht die potentielle Gefahr der Gefäßtraumatisierung durch häufige Katheterwechsel.

EP 0 839 504 A1 beschreibt einen Katheter zum Zuführen eines Stents, wobei der Stent vorgeladen im Katheter vorliegt. Der Katheter ist als sogenannter Over-the-Wire- bzw. OTW-Katheter konstruiert und wird über einen Führungsdraht an den Behandlungsort geschoben. Dabei erstreckt sich der Führungsdraht durch den Katheter und durch den im Katheter angeordneten Stent. Der bekannte Katheter wird durch den darin bereits vorgeladenen Stent versteift, so dass eine Zuführung in enge, stark gewundene Blutgefäße erschwert ist.

Einen ähnlichen Ballonkatheter mit vorgeladenem Stent offenbart US 2007/0225789 A1. Der bekannte Ballonkatheter kann Röntgenmarker aufweisen, die die Position des Stents während der Behandlung sichtbar machen. Die Röntgenmarker sind üblicherweise am Stent selbst angeordnet. Da dieser im Katheter vorgeladen ist, wird gleichzeitig auch eine Positionierung des Katheters im Blutgefäß erkennbar. Eine solche Art der Sichtbarmachung des Katheters ist jedoch für Ballonkatheter zur Zufuhr nicht-vorgeladener Stents ungeeignet. Denn der Ballonkatheter wird in dieser Variante zunächst ohne den Stent in das Blutgefäß geführt. Mangels Röntgenmarkern ist die Positionierung des Ballonkatheters damit erschwert. Der Stent wird nach Befüllen des Ballons in den Ballonkatheter eingeführt und in Richtung des distalen Endes des Katheters vorgeschoben. Sobald der Stent den Ballon erreicht, wird dessen Sichtbarkeit jedoch erheblich reduziert, da der befüllte Ballon die Röntgenmarker des Stents abschattet. Dies erschwert eine genaue und sanfte Entlassung des Stens in ein Blutgefäß.

Der Erfindung liegt die Aufgabe zu Grunde, einen medizinischen Katheter anzugeben, der die Freisetzung selbstexpandierbarer Stents, insbesondere selbstexpandierbarer Stents aus Formgedächtniswerkstoffen ermöglicht, wobei die Platzierung des Stents schnell und unkompliziert erfolgen soll. Erfindungsgemäß wird diese Aufgabe im Hinblick auf den medizinischen Katheter durch den Gegenstand des Anspruchs 1 gelöst.

Die Erfindung beruht auf dem Gedanken, einen medizinischen Katheter zum Zuführen eines selbstexpandierbaren nicht-vorgeladenen Stents in ein Körperhohlorgan anzugeben, wobei der Katheter wenigstens zwei Arbeitskanäle und einen Ballon aufweist, der im distalen Bereich der Arbeitskanäle angeordnet ist. Ein erster Arbeitskanal ist mit dem Ballon fluidverbunden. Die Fluidverbindung ermöglicht die Zufuhr eines gasförmigen und/oder eines flüssigen Fluids, bspw. Luft oder NaCl, oder ein Gemisch davon. Ein zweiter Arbeitskanal mit einer Austrittsöffnung für das Funktionselement erstreckt sich durch den Ballon. Der zweite Arbeitskanal ist mit einem proximal angeordneten, extrakorporalen Anschluss verbunden, der zum Einführen des Funktionselements in den zweiten Arbeitskanal angepasst ist. Der zweite Arbeitskanal weist erfindungsgemäß einen Innendurchmesser auf, der weniger als 0,45 mm beträgt.

Bei dem erfindungsgemäßen Katheter ist außerdem wenigstens ein Röntgenmarker vorgesehen, wobei ein erster Röntgenmarker im Bereich der distalen Austrittsöffnung des zweiten Arbeitskanals, ein zweiter Röntgenmarker im Bereich des distalen Ballonendes und ein dritter Röntgenmarker im Bereich des proximalen Ballonendes angeordnet sind. Damit kann die Position der Katheterspitze bzw. des Ballons bestimmt werden.

Zwar offenbart WO 2004/004821 A1 einen Ballonkatheter für die perkutane transluminale Koronarangioplastie, mit dem Stents implantiert werden können. Zweck des bekannten Ballonkatheters ist es, den Widerstand gegen Knicken des Katheters zu erhöhen, wenn dieser im Körper eines Patienten bewegt wird. Dazu weist der bekannte Ballonkatheter zwei Abschnitte auf, wobei ein proximaler Abschnitt relativ steif und ein distaler Abschnitt relativ biegsam ist (Katheterspitze). Der Ballon ist im Bereich der biegsamen Katheterspitze befestigt. In diesem Bereich weist der Katheter ein Doppellumen auf, wobei ein erstes Inflationslumen mit dem Ballon fluidverbunden ist. Ein zweites Lumen im Bereich der Katheterspitze dient ausschließlich zur Aufnahme eines Führungsdrahtes. Das Lumen für den Führungsdraht weist eine Zugangsöffnung auf, die im Gebrauch des Ballonkatheters intrakorporal angeordnet ist, da sich die Zugangsöffnung im Bereich der Katheterspitze befindet. Der Führungsdraht wird somit im Bereich des starren Katheterabschnittes außerhalb der Katheterleitung geführt und tritt erst im Bereich der Katheterspitze in die Katheterleitung bzw. in das Lumen für den Führungsdraht ein.

Zum Zuführen eines Stents mit dem Ballonkatheter gemäß WO 2004/004821 A1 wird der Stent auf den Außenumfang des deflatierten Ballons gecrimpt, so dass dieser zusammen mit der Katheterspitze entlang des Führungsdrahtes in den Körper eines Patienten eingeführt werden kann. Ist der Stent im Bereich der Stenose platziert, wird der Ballon mit Druck beaufschlagt, so dass der auf dem Ballon sitzende Stent aufgeweitet wird.

Der bekannte Ballonkatheter ist für die Zufuhr von selbstexpandierbaren Stents ungeeignet und dient nur zum Implantieren von ballonexpandierbaren Stents. Im Gegensatz zu dem Ballonkatheter gemäß WO 2004/004821 A1 ist bei der Erfindung der zweite Arbeitskanal für das Funktionselement mit einem Anschluss verbunden, der im Gebrauch des Katheters extrakorporal angeordnet ist. Dadurch wird bewirkt, dass der Katheter außerhalb des Körpers mit einem Stent geladen werden kann, wobei der Stent durch den zweiten Arbeitskanal bis in den Bereich der Stenose bewegt werden kann. Ein extrakorporales Laden des Ballonkatheters gemäß WO 2004/004821 A1 ist hingegen nicht möglich, da der Zugang zum Lumen für den Führungsdraht im Gebrauch intrakorporal angeordnet ist.

Darüber hinaus bietet der erfindungsgemäße Katheter den Vorteil, dass das herkömmliche und vorstehend beschriebene Verfahren zum Freisetzen eines Stents beschleunigt werden kann, da einige Schritte des vorstehend beschriebenen, herkömmlichen Verfahrens entfallen. Insbesondere die Schritte 3, 4 und 6, 7, d.h. das Entfernen des ersten Ballonkatheters, das Einführen des Stent-Delivery-Katheters in die Stenose, das Entfernen des Stent-Delivery-Katheters sowie das Einführen des zweiten Ballonkatheters in die Stenose sind bei der Verwendung des erfindungsgemäßen Katheters nicht erforderlich. Vielmehr kann der selbstexpandierende Stent durch ein Verfahren mit den folgenden Schritten freigesetzt werden:
1. Einführen des erfindungsgemäßen Katheters über einen Führungsdraht in die Stenose;
2. Inflatieren des Ballons zur Weitung der Stenose und anschließendes Deflatieren;
3. Freisetzen des Stents;
4. Inflatieren des Ballons zur Weitung des Stents in der Stenose und anschließendes Deflatieren; und
5. Entfernen des erfindungsgemäßen Katheters.

Aufgrund des erfindungsgemäßen Katheters entfallen somit vier Schritte, wodurch das Verfahren wesentlich beschleunigt wird. Damit verbunden werden die durch die Nutzungszeit von Personal, Operationssälen sowie Geräten, wie beispielsweise Angiographiegeräten, entstehenden Kosten deutlich verringert. Die Kosten von bis zu zwei zusätzlichen Ballonkathetern entfallen vollständig. Überdies entfällt die Gefäßbelastung bei dem im Stand der Technik üblichen Katheterwechseln vollständig, wodurch die Gefäßtraumatisierung, Spasmus und die Schädigung der Endothelzellenschicht minimiert wird. Da kein Katheterwechsel erforderlich ist, wird die Prozedur stark vereinfacht. Der zweite Arbeitskanal kann überdies zur Aspiration während der Aufweitung der Stenose verwendet werden, beispielsweise um abgelöste Gefäßwandpartikel anzusaugen. Dazu kann der zweite Arbeitskanal mit einer Aspirationseinrichtung verbunden sein, wobei der extrakorporale Anschluss entsprechend angepasst ist. Der extrakorporale Anschluss für die Aspirationseinrichtung kann bspw. einen Luer-Anschluss umfassen. Der zweite Arbeitskanal kann ferner zur Zufuhr eines Kontrastmittels verwendet werden, um die Gefäßöffnung nach der Ballondilatation bzw. nach dem Freisetzen des Stents zu kontrollieren.

Bei einer bevorzugten Ausführungsform ist der zweite Arbeitskanal koaxial im ersten Arbeitskanal angeordnet. Die koaxiale Anordnung hat den Vorteil, dass der Katheter relativ flexibel ist. Bei einer weiteren bevorzugten Ausführungsform sind der erste und zweite Arbeitskanal nebeneinander, insbesondere parallel nebeneinander in einer Katheterleitung angeordnet. Die parallele Anordnung der Arbeitskanäle erhöht die Steifigkeit des Katheters. Hingegen wird mit der koaxialen Anordnung der Arbeitskanäle eine Reduktion des Gesamtdurchmessers bzw. Außendurchmessers des Katheters erreicht. Dies gilt insbesondere im Vergleich mit einer parallelen Anordnung der Arbeitskanäle, die jeweils denselben Querschnittsdurchmesser wie in der koaxialen Anordnung aufweisen. Mit anderen Worten weist der Katheter einen kleineren Gesamtdurchmesser auf, wenn die Arbeitskanäle ohne Änderung ihres Querschnittsdurchmessers nicht parallel, sondern koaxial angeordnet sind.

Ein distales Ballonende kann mit einem Außenumfang des zweiten Arbeitskanals verbunden, insbesondere fluiddicht verbunden sein. Dies hat den Vorteil, dass die Abdichtung des Ballons im distalen Bereich kompakt gestaltet ist. Ein proximales Ballonende kann mit dem ersten Arbeitskanal fluidverbunden sein. Diese Ausführungsform ist für zwei Arbeitskanäle, insbesondere für die koaxiale und die parallele Anordnung der beiden Arbeitskanäle geeignet.

Der erste Arbeitskanal kann mit einem proximal angeordneten, extrakorporalen Anschluss verbunden sein, der mit einer Druckeinrichtung verbindbar oder verbunden ist. Der erste und zweite Arbeitskanal können dabei mit einem Mehrfachanschluss, insbesondere mit einem Luer-Konnektor verbunden sein. Damit wird die Handhabung des Katheters erleichtert.

Der zweite Arbeitskanal kann eine reibmindernde Innenbeschichtung für eine translatorische Bewegung des Stents im Arbeitskanal aufweisen. Damit wird die Bewegbarkeit des Funktionselements relativ zum Arbeitskanal verbessert. Die Erfindung wird nachfolgend anhand von Ausführungsbeispielen unter Bezugnahme auf die beigefügten schematischen Zeichnungen mit weiteren Einzelheiten näher erläutert. Darin zeigen
- Fig. 1: einen Längsschnitt durch einen medizinischen Katheter;
- Fig. 2: einen Querschnitt durch ein Ausführungsbeispiel des Katheters mit koaxial angeordneten Arbeitskanälen;
- Fig. 3: einen Querschnitt durch ein Ausführungsbeispiel des Katheters mit nebeneinander angeordneten Arbeitskanälen;
- Fig. 4: einen Längsschnitt durch ein Ausführungsbeispiel des Katheters, bei dem die beiden Arbeitskanäle koaxial angeordnet sind;
- Fig. 5: einen Längsschnitt durch den Katheter gemäß Fig. 4, wobei zusätzlich Röntgenmarker vorgesehen sind; und
- Fig. 6a-6h: eine Darstellung des Ablaufs bei der Dilatation und Implantierung eines Stents mit einer erfindungsgemäßen Vorrichtung.

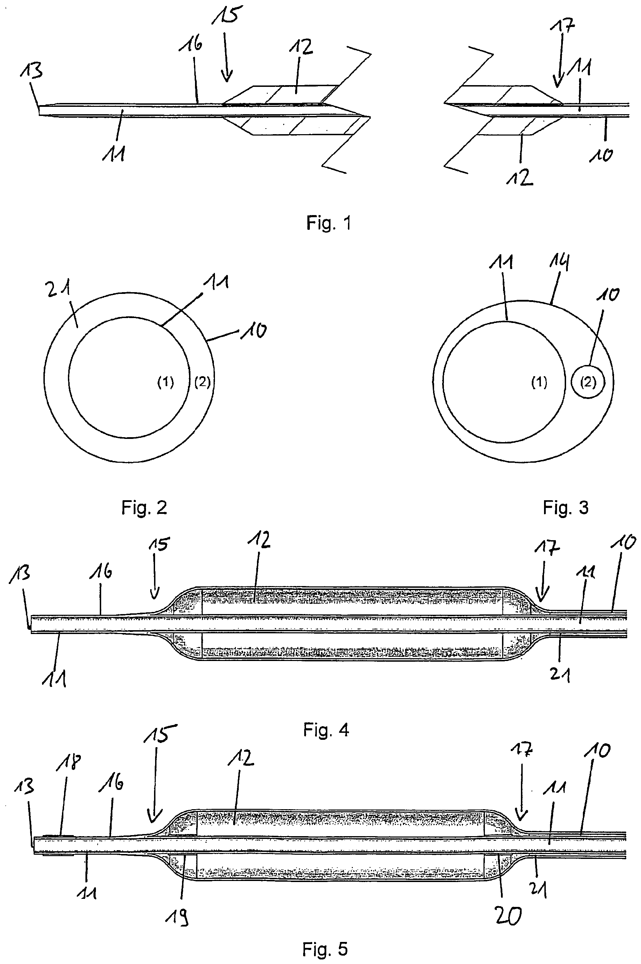

Fig. 1 zeigt ein Ausführungsbeispiel eines medizinischen Katheters zum Zuführen eines Stents in ein Körperhohlorgan. Der Katheter ist zum Zuführen eines selbstexpandierbaren Stents angepasst. Der selbstexpandierbare Stent kann beispielsweise aus einem Formgedächtniswerkstoff hergestellt sein. Die Selbstexpansionseigenschaften können auch anderweitig, beispielsweise mechanisch durch die Rückstellkraft auf Grund einer Federwirkung erreicht werden.

Der Katheter ist zum Zuführen und Freisetzen von selbstexpandierbaren Stents, insbesondere von selbstexpandierbaren Stents aus Formgedächtniswerkstoffen, angepasst.

Der Katheter gemäß Fig. 1 umfasst zwei Arbeitskanäle 10, 11. Es ist auch möglich, mehr als zwei Arbeitskanäle, beispielsweise drei, vier oder mehr als vier Arbeitskanäle vorzusehen.

Der Katheter umfasst ferner einen Ballon 12, der im distalen Bereich der Arbeitskanäle 10, 11 angeordnet ist. Wie in Fig. 1, ebenso wie in Fig. 4, 5 verdeutlicht, ist der Ballon 12 im Bereich der Katheterspitze vorgesehen. Der Ballon 12 ist von der Austrittsöffnung 13 des zweiten Arbeitskanals 11 beabstandet, so dass zwischen der Austrittsöffnung 13 und dem distalen Ballonende 15 ein ballonfreier Abschnitt des zweiten Arbeitskanals 11 ausgebildet ist. Der Ballon 12, insbesondere ein proximales Ende 17 des Ballons 12 ist mit einem ersten Arbeitskanal 10 fluidverbunden, wie in den Fig. 4, 5 zu erkennen. Der Ballon 12 und der erste Arbeitskanal 10 sind im gestreckten Zustand des Katheters fluchtend angeordnet. Zur Verbindung des ersten Arbeitskanals 10 mit dem Ballon 12 ist die Wandung des ersten Arbeitskanals 10 verlängert und geht in die Ballonwandung über. Der Übergang zwischen dem ersten Arbeitskanal 10 und dem Ballon 12 erfolgt durch eine kontinuierliche Durchmesservergrößerung zwischen dem ersten Arbeitskanal 10 und dem maximalen Außenumfang des Ballons 12 im expandierten Zustand (siehe Fig. 4, Fig. 5).

Der erste Arbeitskanal 10 und der Ballon 12 sind einteilig ausgebildet. Es ist auch möglich, den Ballon 12 und den ersten Arbeitskanal 10 zweiteilig auszugestalten und zwischen dem Ballon 12 und dem Arbeitskanal 10 ein zusätzliches Verbindungsstück vorzusehen.

Die Verbindung gemäß Fig. 4, 5 ist insbesondere für die koaxiale Anordnung der beiden Arbeitskanäle 10, 11 gemäß Fig. 2 geeignet. Dabei geht der zwischen den beiden Arbeitskanälen 10, 11 gebildete Ringspalt 21 in das Innenvolumen des Ballons 12 über. Die Verbindung zwischen dem ersten Arbeitskanal 10 und dem Ballon 12 kann anders ausgestaltet sein, beispielsweise wenn die beiden Arbeitskanäle 10, 11 nebeneinander angeordnet sind, wie in Fig. 3 dargestellt. In diesem Fall ist die Verbindung zwischen dem Ballon 12 und dem Arbeitskanal 10 seitlich vom zweiten Arbeitskanal 11 angeordnet (nicht dargestellt).

Der erste Arbeitskanal 10 dient als Steuervolumen zur Versorgung des Ballons 12 mit einem Fluid bzw. zur Abfuhr des Fluids aus dem Ballon 12. Das Fluid kann beispielsweise eine Kochsalzlösung oder steriles Wasser sein. Das Fluid kann auch gasförmig, bspw. Luft sein. In der Praxis ist das Fluid häufig ein Luft/Flüssigkeitsgemisch.

Der Katheter umfasst einen zweiten Arbeitskanal 11 mit einer Austrittsöffnung 13. Die Austrittsöffnung 13 ist distal angeordnet und verbindet das Innenlumen des zweiten Arbeitskanals 11 mit der Umgebung, insbesondere im Gebrauch mit dem Körperhohlorgan, in welchem der Stent freigesetzt wird. Die Austrittsöffnung 13 sowie der Innendurchmesser des zweiten Arbeitskanals 11 ist angepasst derart, dass der Arbeitskanal 11 eine Rückhaltefunktion für den Stent ausübt. Dies bedeutet beispielsweise, dass die Wandung des zweiten Arbeitskanals 11 so stark ist, dass diese die vom selbstexpandierbaren Funktionselement ausgeübten Radialkräfte aufnehmen kann. Ferner ist der zweite Arbeitskanal 11 ausreichend flexibel, so dass die Katheterspitze an relativ enge Gefäßkrümmungen angepasst werden kann.

Wie in den Fig. 1, 4 und 5 zu erkennen, erstreckt sich der zweite Arbeitskanal 11 durch den Ballon 12. Dies bedeutet, dass der Ballon 12 um den Außenumfang 16 des zweiten Arbeitskanals 11 herum angeordnet ist. Der zweite Arbeitskanal 11 und der Ballon 12 sind koaxial angeordnet.

Die Zuordnung des Ballons 12 zum zweiten Arbeitskanal 11, der für die Zufuhr des Stents angepasst ist, hat den Vorteil, dass der Katheter eine Doppelfunktion erfüllt. Zum einen dient der Katheter der Zufuhr des Stents durch den zweiten Arbeitskanal 11. Zum anderen kann mittels des im Bereich der Katheterspitze angeordneten Ballons 12 die erforderliche Dilatation des Gefäßes durchgeführt werden, ohne dass dafür ein Katheterwechsel erforderlich ist. Zur Verwirklichung dieses Prinzips kann es ausreichend sein, dass der zweite Arbeitskanal 11 allgemein dem Ballon 12 zugeordnet ist und zwar im Bereich der Katheterspitze, so dass der Katheter sowohl zum Freisetzen des Stents als auch zum Dilatieren, insbesondere zum Vordilatieren der Stenose sowie zum Nachweiten des implantierten Stents verwendet werden kann. Die symmetrische Anordnung des zweiten Arbeitskanals 11 und des Ballons 12, wie in den Fig. 1, 4 und 5 dargestellt, hat den Vorteil, dass eine einfache radiale Aufweitung sowohl des Gefäßes als auch des implantierten Stents möglich ist, wobei der zweite Arbeitskanal 11 vom Ballon 12 geschützt ist bzw. beim Aufweiten des Ballons 12 nicht mit dem Gefäß zumindest im Bereich des aufgeweiteten Segments bzw. mit dem implantierten Stent in Berührung kommt.

Die Doppelfunktion des Katheters wird dadurch erreicht, dass der zweite Arbeitskanal 11 mit einem proximal angeordneten, im Gebrauch extrakorporalen Anschluss verbunden ist, der zum Einführen des Funktionselements in den zweiten Arbeitskanal 11 angepasst ist. Dies bedeutet praktisch, dass der extrakorporale Anschluss am proximalen Ende der Katheterleitung, also entfernt von der Katheterspitze angeordnet ist. Der extrakorporale Anschluss für den zweiten Arbeitskanal 11 ist somit für den Benutzer direkt zugänglich. Der Anschluss kann beispielsweise zum Laden eines Stents angepasst sein, wobei der Stent vom extrakorporalen Anschluss bis zur Katheterspitze durch den zweiten Arbeitskanal 11 bewegt wird. Der extrakorporale Anschluss für den zweiten Arbeitskanal 11 kann eine an sich bekannte Ladeschleuse für Stents umfassen. Der Anschluss kann beispielsweise einen Luer-Konnektor umfassen.

Der zweite Arbeitskanal 11 für die Zufuhr des Stents 22 erstreckt sich durchgängig von der Katheterspitze zum proximalen Ende des zweiten Arbeitskanals 11 mit der Anschlussöffnung, durch die das der Stent 22 geladen wird. Der zweite Arbeitskanal 11 ist zwischen der Spitze bzw. der Austrittsöffnung und dem der proximalen Anschlussöffnung in radialer Richtung, also seitlich geschlossen.

Durch den im Gebrauch extrakorporal angeordneten Anschluss wird erreicht, dass der Benutzer den Stent laden kann, selbst wenn die Katheterspitze im Bereich des zu behandelnden Blutgefäßes positioniert ist, sich also die Katheterleitung im Wesentlichen vollständig oder zumindest überwiegend im Körper befindet. Es handelt sich also um einen Katheter in OTW Bauweise (over the wire) bei dem sich das Lumen durchgängig vom proximalen zum distalen Ende erstreckt. Im Unterschied dazu weist ein Katheter in RX Bauweise (rapid exchange oder monorail) einen seitlichen Katheterausgang auf, so dass das Lumen vom distalen Ende nur bis zum seitlichen Katheterausgang durchgängig verläuft. Der seitliche Katheterausgang befindet sich dabei relativ nahe am distalen Ende (ca. 15cm vom distalen Ende entfernt).

Ein selbstexpandierbarer Stent kann bei einem Katheter in RX Bauweise nicht durchgeladen werden.

Der selbstexpandierende Stent 22 kann mit dem Transportmittel, insbesondere dem Transportdraht 23 lösbar, verbunden sein. Das Implantat bzw. der Stent kann repositionierbar sein, selbst bei 95%iger Entlassung. Die Abkopplung des Stents 22 kann positionsgesteuert erfolgen. Insbesondere im Bereich des Ballons weist der Katheter ein flexibles Design auf. Hierfür können beispielsweise weiche Ballonmaterialien zum Einsatz kommen, deren Flexibilität größer ist als die Flexibilität von Standard-Ballonmaterialien, wie Nylon oder PET. Konkrete Ballonmaterialien sind PEBAX und Polyurethan. Die Flexibilität des Ballons wird durch die geringe Wandstärke des Ballons unterstützt, die weniger als 0,2 mm betragen kann, insbesondere weniger als 0,15 mm, insbesondere weniger als 0,125 mm, insbesondere weniger als 0,10 mm, insbesondere weniger als 0,075 mm, insbesondere weniger als 0,050 mm, insbesondere weniger als 0,040 mm, insbesondere weniger als 0,030 mm und insbesondere weniger als 0,025 mm. Der Arbeitskanal 11 bzw. der innere Schlauch der sogenannten OTW-Schaft-Konstruktion ist wenigstens zweilagig, insbesondere dreilagig ausgebildet. Der mehrlagige Arbeitskanal 11 ist als Kunststoff-Extrusion ausgebildet und weist die folgenden Eigenschaften auf: hohe Flexibilität, guter Knickschutz, Schiebbarkeit bzw. Pushability, Abriebfestigkeit und Gleitschicht. Der Arbeitskanal 11 weist keine tubular verlaufende Metallverstärkung auf.

Die Katheterlänge beträgt mindestens 1,0 m, insbesondere mindestens 1,25 m, insbesondere mindestens 1,35 m, insbesondere mindestens 1,45 m, insbesondere mindestens 1,5 m, insbesondere mindestens 1,55 m, insbesondere mindestens 1,60 m, insbesondere mindestens 1,65 m. Als Obergrenze kann eine Katheterlänge von 3 m vorgesehen sein. Der Arbeitskanal kann eine Innenschicht bzw. einen Innenliner, insbesondere einen PTFE-Innenliner, zur Minderung der Reibung des Stents 22 aufweisen.

Wie in Fig. 2 dargestellt, ist bei einem Ausführungsbeispiel der zweite Arbeitskanal 11 koaxial im ersten Arbeitskanal 10 angeordnet. Dabei ist ein Ringspalt 21 zwischen den beiden Arbeitskanälen 10, 11 ausgebildet, der als Steuerlumen für den Ballon 12 fungiert. Der zweite Arbeitskanal 11, der im Inneren des ersten Arbeitskanals 10 angeordnet ist, bildet das Hauptlumen, durch das der Stent bewegt wird.

In Fig. 3 ist eine alternative Anordnung der beiden Arbeitskanäle 10, 11 dargestellt, wobei der erste und der zweite Arbeitskanal 11, 12 nebeneinander, insbesondere parallel nebeneinander angeordnet sind. Um die beiden Arbeitskanäle 11, 12 herum ist eine Katheterleitung 14 angeordnet, die die Anordnung der beiden Arbeitskanäle 10, 11 fixiert. Dabei bildet der erste Arbeitskanal 10 das Steuerlumen für den Ballon 12 und der zweite Arbeitskanal 11 das Hauptlumen für die Zufuhr des Stents.

Generell gilt, dass der zweite Arbeitskanal 11 das Hauptlumen und der erste Arbeitskanal 10 das Steuerlumen bilden. Der Durchmesser des Hauptlumens ist größer als der Durchmesser des Steuerlumens.

In den Fig. 4, 5 ist dargestellt, dass das distale Ballonende 15 mit dem Außenumfang 16 des zweiten Arbeitskanals 11 fluiddicht verbunden ist. Das proximale Ballonende 17 ist mit dem ersten Arbeitskanal 10 fluidverbunden. Damit schließt der Ballon 12 einerseits fluiddicht mit dem zweiten Arbeitskanal 11 ab und ist andererseits durch den ersten Arbeitskanal 10 inflatierbar bzw. deflatierbar.

Der als Steuerlumen ausgebildete erste Arbeitskanal 10 ist mit einem proximal angeordneten, im Gebrauch extrakorporalen Anschluss verbunden. Im Zusammenhang mit dem extrakorporalen Anschluss für den zweiten Arbeitskanal 11 ist ein Mehrfachanschluss, beispielsweise ein Y-Luer-Konnektor möglich. Der Konnektor bzw. Anschluss für den ersten Arbeitskanal 10 ist entweder fest oder lösbar mit einer Druckeinrichtung verbunden. Die Druckeinrichtung ist ausgebildet zur Erzeugung eines Überdrucks zum Inflatieren bzw. zur Erzeugung eines Unterdrucks zum Deflatieren des Ballons 12. Die Druckeinrichtung kann beispielsweise eine Spritze umfassen. Andere Druckeinrichtungen sind möglich.

Der zweite Arbeitskanal 11 ist mit einer reibmindernden Innenbeschichtung für eine translatorische Bewegung des Stents im Arbeitskanal 11 versehen. Als Beschichtungen kommen beispielsweise PTFE oder reibmindernde Oberflächenmodifizierungen in Frage. Andere Materialien für die Beschichtung sind ebenfalls möglich.

In Fig. 5 ist dargestellt, dass die Katheterspitze mehrere Röntgenmarker 18, 19, 20 aufweist. Ein erster Röntgenmarker 18 ist im Bereich der distalen Austrittsöffnung 13 angeordnet und dient zur Lokalisation des Endes der Katheterspitze. Ein zweiter Röntgenmarker 19 ist im Bereich des distalen Ballonendes 15 angeordnet. Ein dritter Röntgenmarker 20 ist im Bereich des proximalen Ballonendes 17 angeordnet. Der zweite und dritte Röntgenmarker 19, 20 dienen dazu, die Position des Ballons festzustellen.

Der medizinische Katheter wird an sich, d.h. ohne Peripheriegeräte offenbart und beansprucht und umfasst im Wesentlichen die mehrlumige Katheterleitung mit den beiden extrakorporalen Anschlüssen für den ersten Arbeitskanal 10 und den zweiten Arbeitskanal 11.

Konkrete Dimensionsbeispiele für den Katheter umfassen Ballondurchmesser von 1,5-4,5 mm, Ballonlängen von 20-150 mm sowie Ballons mit einer Druckstabilität bis 20 bar. Der Katheterschaftdurchmesser kann kleiner 4 Fr, d.h. ≤ 1,32 mm sein. Als Untergrenze für den Katheterschaftdurchmesser können 1,15 mm oder 1,0 mm oder ≤ 1,0 mm in Frage kommen. Das Innenlumen kann z.B. 0,5 mm oder 0,7 mm betragen. Konkret kann der Innendurchmesser des zweiten Arbeitskanals 11 bzw. des Hauptlumens ≤ 1,0 mm, insbesondere ≤ 0,9 mm, ≤ 0,8 mm, ≤ 0,7 mm, ≤ 0,68 mm, ≤ 0,65 mm, ≤ 0,6 mm, ≤ 0,55 mm, ≤ 0,5 mm, ≤ 0,45 mm, ≤ 0,4 mm, ≤ 0,35 mm betragen. Die Ballonlänge kann ≤ 210 mm, insbesondere ≤ 150 mm, ≤ 125 mm, ≤ 100 mm, ≤ 80 mm, ≤ 40 mm, ≤ 30 mm betragen. Der Ballondurchmesser kann ≤ 6,0 mm, insbesondere ≤ 5,5 mm, ≤ 5,0 mm, ≤ 4,5 mm, ≤ 4,0 mm, ≤ 3,5 mm, ≤ 3,0 mm, ≤ 2,5 mm, ≤ 2,0 mm, ≤ 1,5 mm betragen. Der Außendurchmesser des Katheterschafts kann ≤ 2,0 mm, insbesondere 1,75 mm, ≤ 1,5 mm, ≤ 1,4 mm, ≤ 1,3 mm, ≤ 1,25 mm, ≤ 1,20 mm, ≤ 1,15 mm, ≤ 1,10 mm, ≤ 1,05 mm, ≤ 1,00 mm, ≤ 0,85 mm, ≤ 0,90 mm, ≤ 0,85 mm, ≤ 0,80 mm betragen. Der Durchmesser des ersten Arbeitskanals bzw. des Steuerlumens für den Ballon 12 kann ≤ 1,0 mm, insbesondere ≤ 0,75 mm, insbesondere ≤ 0,5 mm, ≤ 0,4 mm, ≤ 0,3 mm, ≤ 0,25 mm, ≤ 0,20 mm, ≤ 0,15 mm, ≤ 0,125 mm, ≤ 0,10 mm, ≤ 0,09 mm, ≤ 0,08 mm, ≤ 0,07, ≤ 0,06 mm, ≤ 0,05 mm betragen.

Die Katheterspitze kann atraumatisch und/oder flexibel ausgebildet sein.

Als Materialien für den Katheter eignen sich Kunststoffe, Metalle, Formgedächtnismaterialien, wie Nitinol, sowie röntgensichtbare Materialien.

Der Katheter ermöglicht ferner die Aspiration während oder nach der Aufweitung der Stenose durch das Hauptlumen. Dazu ist der zweite Arbeitskanal 11 mit einer Absaugeinrichtung verbunden bzw. verbindbar. Dies hat den Vorteil, dass bei dem Aufweiten abgelöste Gefäßwandpartikel durch den zweiten Arbeitskanal 11 abgesaugt werden können.

Ein weiterer Vorteil des Katheters besteht darin, dass der zweite Arbeitskanal 11 beim Aufblasen des Ballons nicht kollabiert. Ferner ist es möglich, durch den zweiten Arbeitskanal 11 ein Kontrastmittel zu injizieren. Konkret kann nach dem Dilatieren der Stenose der zweite Arbeitskanal 11 des Katheters zur Kontrastmittelgabe verwendet werden, um zu prüfen ob die Stenose geöffnet wurde. Dazu ist der zweite Arbeitskanal 11 mit einer entsprechenden Einrichtung zur Injektion eines Kontrastmittels, beispielsweise einer Spritze verbindbar oder verbunden. Der Katheter hat ferner den Vorteil, dass durch einen einzigen Katheter mehrere Stenosen aufgedehnt und/oder mehrere Stents freigesetzt werden können.

Der mehrlumige Katheter ist für closed-cell-stents, die von der proximalen Katheterseite geladen werden geeignet. Die Stents sind vorzugsweise aus einem Formgedächtnismaterial, beispielsweise aus Nitinol, hergestellt. Die closed-cell-stents sind mit dem Katheter repositionierbar, bis diese komplett freigesetzt werden. Bei nicht-vorgeladenen Stents, die während des Eingriffs von der proximalen Katheterseite geladen werden, kann der Katheter ohne Stent über den Führungsdraht geschoben und im Gefäß positioniert werden. Daraufhin wird der Führungsdraht entfernt, wobei der Katheter an Ort und Stelle verbleibt, und der Stent wird mit einem Pusher durch den zweiten Arbeitskanal 11 bewegt.

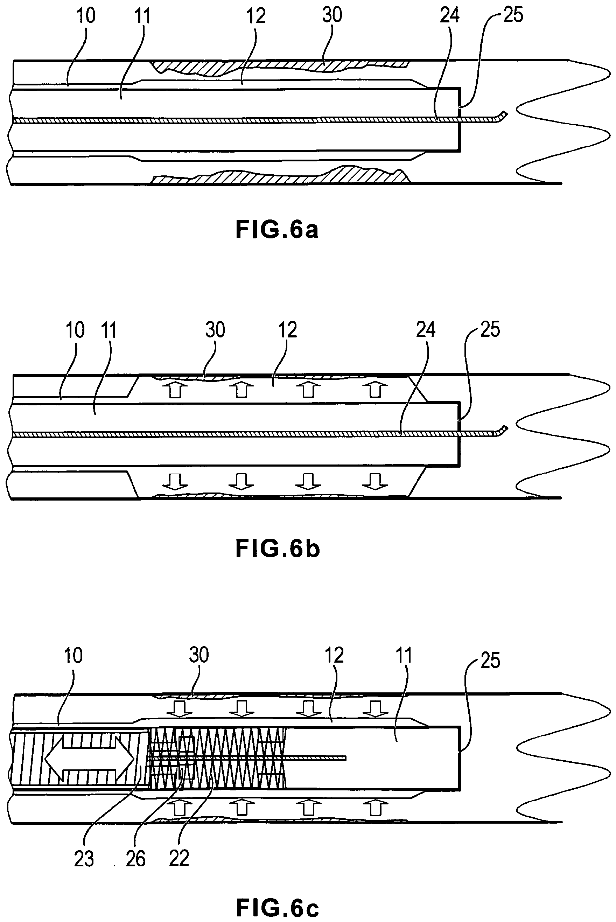

Der Katheter wird wie folgt gebraucht:
Wie in Fig. 6a dargestellt, wird der Katheter über einen Führungsdraht 24 an die zu behandelnde Stelle, insbesondere die Stenose 30 manövriert. Die Katheterspitze 25 befindet sich etwas distal von der Stenose 30. Der Ballon 12 wird in etwa auf Höhe der Stenose 30 positioniert. Wie in Fig. 6b dargestellt, wird die Stenose 30 mit dem Ballon 12 vordilatiert. Der Ballon wird anschließend deflatiert. In Fig. 6c ist dargestellt, dass das Implantat, insbesondere ein Stent, der durch eine Schleuse vom proximalen Ende des Arbeitskanals 11 in den Katheter geladen wurde, bis zum distalen Ende des Arbeitskanals 11 transportiert wird. Dabei ist die Verbindung zwischen dem Stent 22 und dem Transportdraht 23 so ausgebildet, dass der Stent 22 in proximaler und distaler Richtung verschoben werden kann. Hierzu weist der Stent 22 bzw. der Stent am proximalen und distalen Ende jeweils nach innen ragende Anschläge auf. Am Transportdraht 23 ist ein entsprechendes Gegenstück 26 befestigt, das sich radial nach außen erstreckt und jeweils mit dem proximalen bzw. distalen Anschlag in Berührung kommt, um eine in proximaler Richtung bzw. distaler Richtung wirkende Axialkraft zu übertragen.

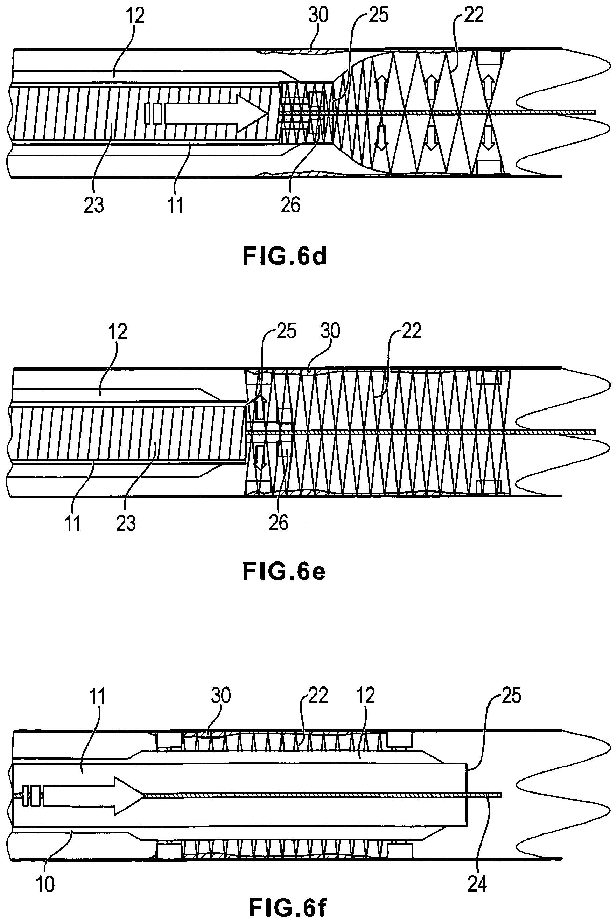

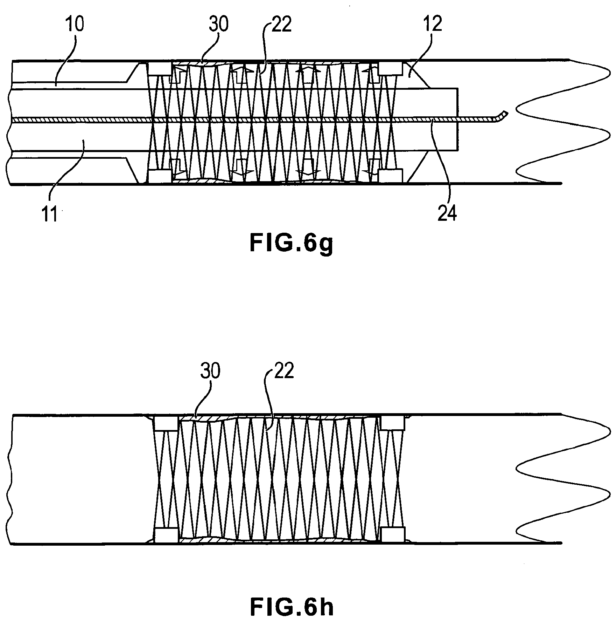

Wie in Fig. 6d dargestellt, wird der Stent 22 im Bereich der zuvor dilatierten Stenose 30 freigesetzt. Durch die positionsgesteuerte Abkoppelfunktion des Funktionselements lässt sich die Position des Implantats, falls erforderlich, verändern. Dazu wird das Implantat in den Arbeitskanal 11 wieder eingezogen. Das Implantat ist also wieder einziehbar. Wie in Fig. 6e dargestellt, wird das Implantat komplett freigesetzt und vom Transportdraht 23 abgekoppelt, wenn die korrekte Position des Implantats vorliegt. Dazu wird der Katheter relativ zum Implantat zurückgezogen. Nach dem Freisetzen des Implantats kann der Katheter im Lumen des Implantats positioniert werden, um das Implantat bzw. den Stent nachzudilatieren. Dieser Vorgang ist in Fig. 6f sowie in Fig. 6g dargestellt. Die Möglichkeit des Nachdilatierens hat den Vorteil, dass der Stent gefäßschonend mit vergleichsweise geringer Radialkraft ausgelegt sein kann. Die Radialkraft kann also zur Aufbringung der Haltekraft ausreichen, muss aber nicht die zur Aufweitung der Stenose erforderliche Expansionskraft aufbringen. Dies erfolgt zumindest teilweise durch die Nachdilatation mittels des erfindungsgemäßen Katheters.

In Fig. 6h ist das selbstexpandierte Implantat bzw. das durch die Ballondilatation unterstützte selbstexpandierte Implantat dargestellt, das die Stenose offenhält. Alle vorangegangenen Schritte wurden mit einem einzigen Katheter, nämlich dem erfindungsgemäßen Katheter ausgeführt.

Als Einsatzgebiet kommt vorzugsweise BTK (Below The Knee) in Frage. Höhere bzw. tiefere Körperbereiche sind auch denkbar. Ferner ist der Katheter bspw. in der Kardiologie und Neuroradiologie bzw. Neurochirurgie einsetzbar.

### Bezugszeichenliste

- 10: erster Arbeitskanal
- 11: zweiter Arbeitskanal
- 12: Ballon
- 13: Austrittsöffnung
- 14: Katheterleitung
- 15: distales Ballonende
- 16: Außenumfang
- 17: proximales Ballonende
- 18: erster Röntgenmarker
- 19: zweiter Röntgenmarker
- 20: dritter Röntgenmarker
- 21: Ringspalt
- 22: Stent
- 23: Transportdraht
- 24: Führungsdraht
- 25: Katheterspitze
- 26: Gegenstück
- 30: Stenose

## Patentansprüche

1. Medizinischer Katheter zum Zuführen eines selbstexpandierbaren, nicht-vorgeladenen Stents in ein Körperhohlorgan mit wenigstens zwei Arbeitskanälen (10, 11) und einem Ballon (12), der im distalen Bereich der Arbeitskanäle (10, 11) angeordnet ist,
wobei
- ein erster Arbeitskanal (10) mit dem Ballon (12) fluidverbunden ist und ein zweiter Arbeitskanal (11) sich durch den Ballon (12) erstreckt,
- der zweite Arbeitskanal (11) eine distale Austrittsöffnung (13) für den Stent aufweist und mit einem proximal angeordneten, im Gebrauch extrakorporalen Anschluss verbunden ist, der zum Einführen des Stents in den zweiten Arbeitskanal (11) angepasst ist,
- der Innendurchmesser des zweiten Arbeitskanals (11) kleiner 0,45 mm beträgt, und
- wenigstens ein Röntgenmarker (18, 19, 20) vorgesehen ist,
und wobei
ein erster Röntgenmarker (18) im Bereich der distalen Austrittsöffnung (13) des zweiten Arbeitskanals (11), ein zweiter Röntgenmarker (19) im Bereich des distalen Ballonendes (15) und ein dritter Röntgenmarker (20) im Bereich des proximalen Ballonendes (17) angeordnet sind.

2. Katheter nach Anspruch 1,
**dadurch gekennzeichnet, dass**
der zweite Arbeitskanal (11) koaxial im ersten Arbeitskanal (10) angeordnet ist.

3. Katheter nach Anspruch 1,
**dadurch gekennzeichnet, dass**
der erste und zweite Arbeitskanal (11, 12) nebeneinander, insbesondere parallel nebeneinander in einer Katheterleitung (14) angeordnet sind.

4. Katheter nach wenigstens einem der Ansprüche 1-3,
**dadurch gekennzeichnet, dass**
ein distales Ballonende (15) mit einem Außenumfang (16) des zweiten Arbeitskanals (11) verbunden, insbesondere fluiddicht verbunden ist.

5. Katheter nach wenigstens einem der Ansprüche 1-4,
**dadurch gekennzeichnet, dass**
ein proximales Ballonende (17) mit dem ersten Arbeitskanal (10) fluidverbunden ist.

6. Katheter nach wenigstens einem der Ansprüche 1-5,
**dadurch gekennzeichnet, dass**
der erste Arbeitskanal (10) mit einem proximal angeordneten, extrakorporalen Anschluss verbunden ist, der mit einer Druckeinrichtung verbindbar oder verbunden ist.

7. Katheter nach wenigstens einem der Ansprüche 1-6,
**dadurch gekennzeichnet, dass**
der erste und zweite Arbeitskanal (10, 11) mit einem Mehrfachanschluss, insbesondere einem Luer-Konnektor verbunden sind.

8. Katheter nach wenigstens einem der Ansprüche 1-7,
**dadurch gekennzeichnet, dass**
der zweite Arbeitskanal (11) eine reibmindernde Innenbeschichtung für eine translatorische Bewegung des Funktionselements im zweiten Arbeitskanal (11) aufweist.

## Claims

1. A medical catheter for supplying a self-expandable non-preloaded stent into a body cavity using at least two working channels (10, 11) and one balloon (12), which is arranged in the distal region of the working channels (10, 11),
wherein
- a first working channel (10) is fluidically connected to the balloon (12), and a second working channel (11) extends through the balloon (12),
- the second working channel (11) has a distal outlet opening (13) for the stent and is connected to an extracorporeal connection, which is arranged proximally during use and is adapted for insertion of the stent into the second working channel (11),
- the inside diameter of the second working channel (11) is less than 0.45 mm, and
- at least one X-ray marker (18, 19, 20) is provided,
and wherein
a first X-ray marker (18) is arranged in the area of the distal outlet opening (13) of the second working channel (11), a second X-ray marker (19) is arranged in the area of the distal balloon end (15), and a third X-ray marker (20) is arranged in the area of the proximal balloon end (17).

2. The catheter according to claim 1,
**characterized in that**
the second working channel (11) is arranged coaxially in the first working channel (10).

3. The catheter according to claim 1,
**characterized in that**
the first and second working channels (11, 12) are arranged side by side, in particular in parallel to one another in a catheter line (14).

4. The catheter according to at least one of claims 1 to 3,
**characterized in that**
a distal balloon end (15) is connected to an outside circumference (16) of the second working channel (11), in particular in a fluid-tight connection.

5. The catheter according to at least one of claims 1 to 4,
**characterized in that**
a proximal balloon end (17) is fluidically connected to the first working channel (10).

6. The catheter according to at least one of claims 1 to 5,
**characterized in that**
the first working channel (10) is connected to an extracorporeal connection arranged proximally and connected or connectable to a pressure device.

7. The catheter according to at least one of claims 1 to 6,
**characterized in that**
the first and second working channels (10, 11) are connected to a multiple connection, in particular a Luer connector.

8. The catheter according to at least one of claims 1 to 7,
**characterized in that**
the second working channel (11) has a friction-reducing inside coating for a translational movement of the function element in the second working channel (11).

## Revendications

1. Cathéter médical pour conduire un stent auto-extensible non-préchargé dans un organe de corps creux comprenant au moins deux canaux de travail (10, 11) et un ballonnet (12) qui est disposé dans la partie distale des canaux de travail (10, 11), dans lequel
- un premier canal de travail (10) est relié fluidiquement au ballonnet (12) et un second canal de travail (11) s'étend à travers le ballonnet (12),
- le second canal de travail (11) présente une ouverture de sortie distale (13) pour le stent et est relié à un raccord extracorporel en utilisation, disposé de façon proximale, qui est adapté pour introduire le stent dans le second canal de travail (11),
- le diamètre intérieur du second canal de travail (11) est inférieur à 0,45 mm, et
- au moins un marqueur radiographique (18, 19, 20) est prévu,
et dans lequel
sont disposés un premier marqueur radiographique (18) au niveau de l'ouverture de sortie distale (13) du second canal de travail (11), un deuxième marqueur radiographique (19) au niveau de l'extrémité de ballonnet distale (15) et un troisième marqueur radiographique (20) au niveau de l'extrémité de ballonnet proximale (17).

2. Cathéter selon la revendication 1, **caractérisé en ce que** le second canal de travail (11) est disposé coaxialement dans le premier canal de travail (10).

3. Cathéter selon la revendication 1, **caractérisé en ce que** les premier et second canaux de travail (11, 12) sont disposés l'un à coté de l'autre, en particulier parallèles l'un à l'autre, dans une ligne de cathéter (14) .

4. Cathéter selon au moins l'une des revendications 1 - 3, **caractérisé en ce qu'**une extrémité de ballonnet distale (15) est reliée, en particulier en étanchéité fluidique, à un pourtour extérieur (16) du second canal de travail (11).

5. Cathéter selon au moins l'une des revendications 1 - 4, **caractérisé en ce qu'**une extrémité de ballonnet proximale (17) est reliée de façon fluidique au premier canal de travail (10).

6. Cathéter selon au moins l'une des revendications 1 - 5, **caractérisé en ce que** le premier canal de travail (10) est relié à un raccord extracorporel, disposé de façon proximale, qui peut être relié ou est relié à un dispositif de pression.

7. Cathéter selon au moins l'une des revendications 1 - 6, **caractérisé en ce que** le premier et le second canaux de travail (10, 11) sont connectés à un raccord multiple, en particulier un connecteur Luer.

8. Cathéter selon au moins l'une des revendications 1 - 7, **caractérisé en ce que** le second canal de travail (11) présente un revêtement intérieur réduisant le frottement pour un déplacement en translation de l'élément fonctionnel dans le second canal de travail (11).
